# EUROPEAN PATENT SPECIFICATION

(11) **EP 4 289 467 B1**
(45) Date of publication and mention of the grant of the patent: **19.08.2026**
(21) Application number: 23191401.1
(22) Date of filing: 16.04.2014
(51) Int. Cl.: A61F 2/00, A61B 17/00, A61F 2/02, A61L 27/36, A61K 9/00, A61M 31/00

(54) **INSTRUMENTS FOR USE WITH IMPLANTABLE ENCAPSULATION DEVICES**
INSTRUMENTE ZUR VERWENDUNG MIT IMPLANTIERBAREN VERKAPSELUNGSVORRICHTUNGEN
INSTRUMENTS DESTINÉS À ÊTRE UTILISÉS AVEC DES DISPOSITIFS D'ENCAPSULATION IMPLANTABLES

(43) Date of publication of application: 13.12.2023
(62) Document number(s) of the earlier application(s) in accordance with Art. 76 EPC: 14725332.2 / 3 131 497
(73) Proprietor: ViaCyte, Inc., San Diego, CA 92121 (US)
(72) Inventor: SO, Vincent, San Diego, CA 92126 (US); OLSON, Erik, Escondido, CA 92025 (US); SCOTT, Michael, San Diego, CA 92121 (US); GREEN, Chad, San Diego, CA 92131 (US); STROLLO, Giacomo, San Diego, CA 92101-1252 (US); PRADO, Gustavo, San Diego, CA 92103 (US); MCGREEVY, Craig, La Jolla, CA 92037 (US); MARTINSON, Laura, San Diego, CA 92104 (US); KOENIG, Donald, San Diego, CA 92103 (US)
(74) Representative: Vossius & Partner Patentanwälte Rechtsanwälte mbB

(56) References cited:
- WO-A2-01/08630
- US-A- 5 964 261

## Description

### STATEMENT OF GOVERNMENT SUPPORT

This research was made possible, in part, by an award from the California Institute for Regenerative Medicine (Award No. DR1-01423). The contents of this publication are solely the responsibility of the inventors and do not necessarily represent the official views of CIRM or any other agency of the state of California.

### BACKGROUND OF THE INVENTION

### 1. Field of Invention

The present invention relates generally to a cellular therapy and means and methods for holding, transferring, sizing, marking and deploying a cell encapsulation implantable device for the treatment of human diseases, specifically, diabetes mellitus.

### 2. Description of Related Art

Cell replacement therapy for certain diseases can be therapeutically treated by transferring cells, tissues, or organs into a patient having the particular disease. The main hurdles to a commercial cell therapy remain a renewable cell source and an encapsulation source which provides allo-protection against host immunity. Ideally, such an implantable device minimizes or eliminates patient use of long term immune-suppressive drugs.

Previously, Applicants have described both a renewable cell source and macro-encapsulation drug delivery system suitable for at least the purpose of pancreatic progenitor cell delivery for production of insulin *in vivo* in response to glucose stimulation. See, for example, at least U.S. Application Serial Nos. 12/099,759, entitled METHODS OF PRODUCING PANCREATIC HORMONES, filed April 8, 2008; 12/618,659, entitled ENCAPSULATION OF PANCREATIC LINEAGE CELLS DERIVED FROM HUMAN PLURIPOTENT STEM CELLS, filed November 13, 2009; 14/106,330, entitled IN VITRO DIFFERENTIATION OF PLURIPOTENT STEM CELLS TO PANCREATIC ENDODERM CELLS (PEC) AND IMMATURE BETA CELLS, filed December 12, 2013; 14/201,630, filed March 7, 2014; and PCT/US2014/026529, IN VITRO DIFFERENTIATION OF PLURIPOTENT STEM CELLS TO PANCREATIC ENDODERM CELLS (PEC) AND ENDOCRINE CELLS, filed March 13, 2014; PCT/US2014/022109, 3-DIMENSIONAL LARGE CAPACITY CELL ENCAPSULATION DEVICE, filed March 7, 2014; and U.S. Design Application Numbers: 29/408,366 filed December 12, 2011; 29/408,368 filed December 12, 2011; 29/423,365 filed May 31, 2012; 29/447,944 filed March 13, 2013; 29/484,363, 29/484,359, 29/484,360, 29/484,357;29/484,356, 29/484,355, 29/484,362 and 29/484,35, titled 3-DIMENSIONAL LARGE CAPACITY CELL ENCAPSULATION DEVICE and filed March 7, 2014.

Such an encapsulated cell product ("combination product") for treatment of diabetes is not commercially available. A commercial combination product should provide systems and methods for securing and maintaining the integrity of an implantable device in its own case, an assembly for sterilizing the implantable device and case, filling the implantable device with living cells in an aseptic manner, storing and shipping the combination product to a clinical site, preparing an anatomical implantation site by sizing it and marking the anatomical implantation site, then holding, transferring and deploying the combination product to the anatomical implantation site. A pouch for an implantable cell container is disclosed in US 5964261 A.

### SUMMARY OF THE INVENTION

The invention is a device-fill pouch assembly according to claim 1.

Disclosed herein is a method of deploying an implantable device for a cell therapy, the method comprising the steps of positioning at an anatomical implantation site an implantable device and an assembly comprising a holder having a first and second plate and a deployer plate, wherein the second holder further comprises a rod and handle, wherein the deployer element is connected to the rod and is capable of moving along the axis of the rod; and moving the deployer element relative to rod and first and second holder; thereby deploying or delivering the implantable device at the anatomical implantation site. Also disclosed is an assembly for deploying an implantable device, the assembly comprising a first holder; a second holder comprising a rod or shaft and a handle; and a deployer mechanism between said first and second holder; wherein the deployer mechanism is adapted to effect deployment of an implantable device; wherein the first holder and deployer mechanism are detachably connected to the second holder rod and is capable of movement along the long axis of the rod; and wherein movement of the deployer mechanism relative to the first and second holder delivers or deploys the implantable device. Also disclosed is a case for holding an implantable planar-shape device, the case comprising: a detachably connected cover body and base body, wherein the cover body has a first latch portion and the base body has a second latch portion configured to secure the cover body to the base body when the case is in a closed position, and wherein the cover body and the base body when secured comprise a volume having at least one opening to allow passage of one or more biologically active materials therethrough. Additional embodiments are disclosed.

The foregoing, and other features and advantages of the invention will be apparent from the following, more particular description of the preferred embodiments of the invention, the accompanying drawings, and the claims.

### BRIEF DESCRIPTION OF THE DRAWINGS

For a more complete understanding of the embodiments of the invention, and the advantages thereof, reference is now made to the ensuing descriptions taken in connection with the accompanying drawings briefly described as follows:
**FIG. 1** illustrates an exploded perspective view of a device case to show the component parts and an implantable device according to an embodiment of the invention;
**FIG. 2** illustrates a perspective view of an open device case of the invention as illustrated in FIG. 1 having a base body and cover body attachable at the hinge and containing an implantable device with a port therein;
**FIG. 3** illustrates a perspective view of a closed device case of the invention as illustrated in FIG. 1 with the base body and cover body attachable at the hinge and containing an implantable device with a port therein;
**FIGS. 4A-4E** illustrate a perspective views of finger grip configurations according to embodiments of the invention;
**FIG. 5** illustrates a top view of window configurations on the cover body and base body of a device case according to an embodiment of the invention;
**FIG. 6** illustrates a perspective view of a device fill pouch assembly (DFPA) for housing and sterilizing the device case and an implantable device therein according to an embodiment of the invention;
**FIG. 7** illustrates a perspective view of a device case storage container according to an embodiment of the invention;
**FIG. 8** illustrates an exploded perspective view of the FIG. 7 device case storage container and components for inserting the device case and implantable device therein, with the cover sheet of the container removed for clarity of illustration, according to an embodiment of the invention;
**FIG. 9** illustrates a perspective view of a device case storage container with an implantable device case and implantable device therein, with the cover sheet of the container removed for clarity of illustration, according to an embodiment of the invention;
**FIG. 10** illustrates a perspective view of a device case storage container shipping bag, with the cover sheet of the container removed for clarity of illustration, according to an embodiment of the invention;
**FIG. 11** illustrates a perspective view of a device case storage container shipping bag with a device case storage container therein, with the cover sheet of the container removed for clarity of illustration, according an embodiment of the invention;
**FIGS. 12A-12B** illustrate perspective views of a surgical sizer, outside and inside the implant site according to an embodiment of the invention;
**FIGS. 13A-13I** illustrate exploded perspective views of, and sequences of using, an implantable device deployer (FIG.13A), a implantable device deployer to show the component parts (FIG. 13B), during transfer of the implantable device from the device case (FIG.13C-E), delivery and deployment of the implantable device at the implant site (FIG. 13F-H), and a cross-section of the shaft of the deployer (FIG.13I) ; and
**FIGS. 14A-14C** illustrate perspective views of an alternative implantable device deployer embodiment (FIG. 14A), an exploded perspective view of the implantable device deployer to show the component parts thereof (FIG. 14B), and after delivery and deployment of the implantable device at the anatomical implant site (FIG.14C) according an embodiment of the invention.

### DETAILED DESCRIPTION OF EMBODIMENTS

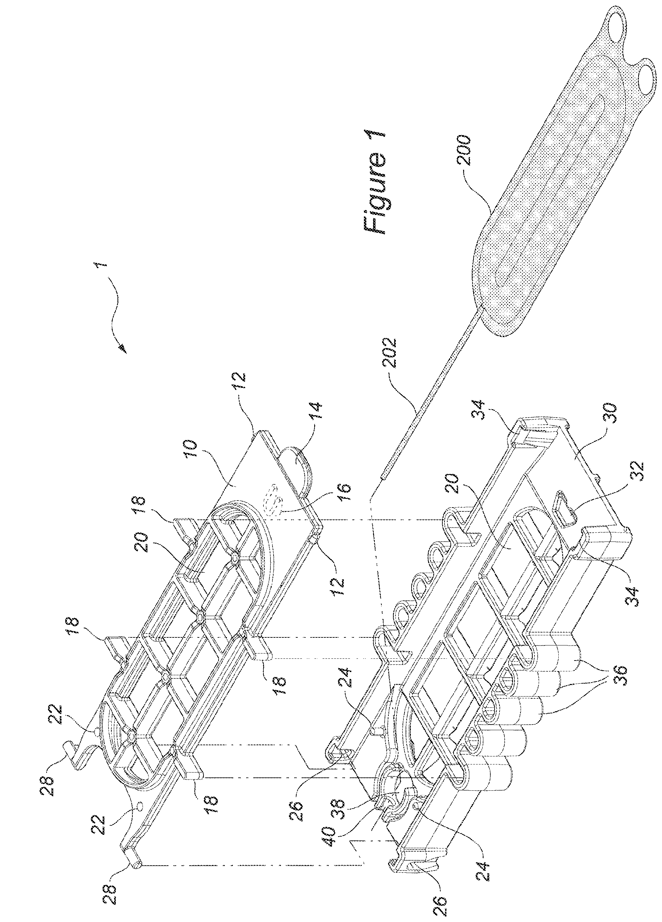

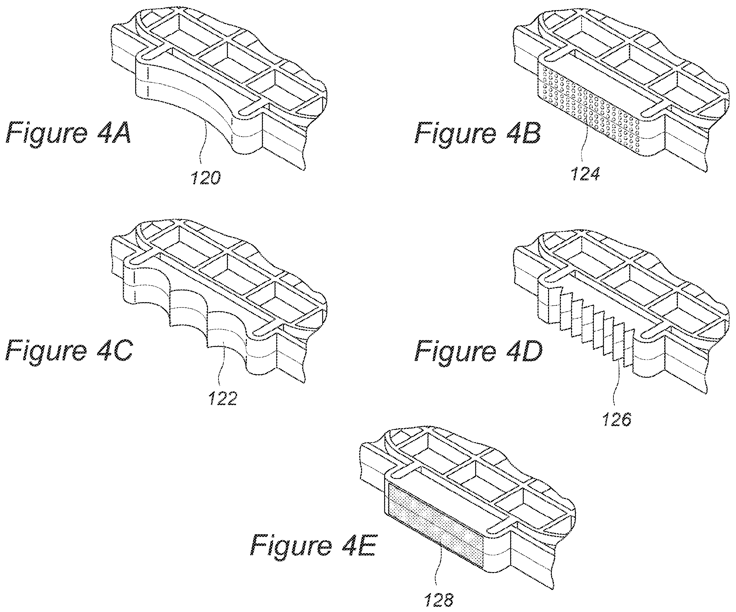

Further features and advantages of the invention, as well as the structure and operation of various embodiments of the invention, are described in detail below with reference to the accompanying **FIGS. 1-14****,** wherein like reference numerals refer to like elements. Although embodiments of the invention are described in the context of implantable devices with pancreatic progenitor cells for the treatment of diabetes mellitus, one of ordinary skill in the art readily appreciates that the present invention is applicable for macro-encapsulation of any type of living cells, therapeutic agents, or mixtures thereof, including but not limited to thyroid cells, parathyroid cells, pancreatic cells, intestinal cells, thymus cells, hepatic cells, endocrine cells, skin cells, hematopoietic cells, bone marrow stem cells, renal cells, muscle cells, neural cells, stem cells, embryonic stem cells, lineage-restricted cells, progenitor cells, precursor cells, genetically engineered cells, tumor cells, and derivatives and combinations thereof for the treatment of one or more disease or disorder, including, but not limited to diabetes mellitus. Also contemplated are cells producing cell-based products such as proteins (e.g. hormones and/or other proteins deficient in human diseases and the like), antibodies, antibiotics, lymphokines and the like for therapeutic indications. One of ordinary skill in the art also appreciates that the present invention is applicable to different implantable device types, materials, sizes, and/or configurations.

Various publications including scientific journal articles, patent publications and patents are herein referred to and the disclosure of each of them is incorporated herein by reference in its entirety. For example, methods of making insulin-producing cells and the implantation of implantable devices with pancreatic progenitors derived from human pluripotent stem cells for the production of insulin-producing cells are disclosed in Applicants U.S. Patent Nos.: 7,534,608; 7,695,965; 7,993,920; 8,338,170; 8,278,106; and 8,425,928. In the following the unit 1 psi corresponds to 6.89 kPa. The unit 1 mil corresponds to 0.0254 mm.

### Device Case

**FIGS. 1-4** illustrate nonlimiting, nonexclusive embodiments of a case **1** to secure and maintain the integrity of an implantable device. The case is also referred to an "implantable device case", "device case", "cage", "case" or equivalents thereof. For example, in one embodiment the device case maintains the integrity of the implantable device when the implantable device is sterilized or sterilizable (e.g. autoclave-safe), transferred, filled and the like. The case is generally a case **1** that comprises a first portion (e.g., a lid, cover, cover body, top body) **10,** a second portion (e.g., bottom, base, tray, base tray, receptacle body) **30,** a third portion (e.g., windows, openings, partitions, sections) **20,** a fourth portion (a grippable means e.g. finger grips, handle, side walls) **36,** a fifth portion (e.g., a hinge system) **26, 28,** a sixth portion (a lock, latch or closing or attachment system e.g. latch or snap enclosures) **16, 32,** seventh portion (a port sealing area or port sealing stage area) **38,** and an eight portion (e.g. a ramp and/or side rails to accommodate and guide the deployer). The case **1** is generally configured to house an implantable device **200** therein, but can be configured to house any device, instrument, component, apparatus, element, material and the like therein with the purpose of securing and maintain the integrity of such device, instrument, component, apparatus, element, material and the like.

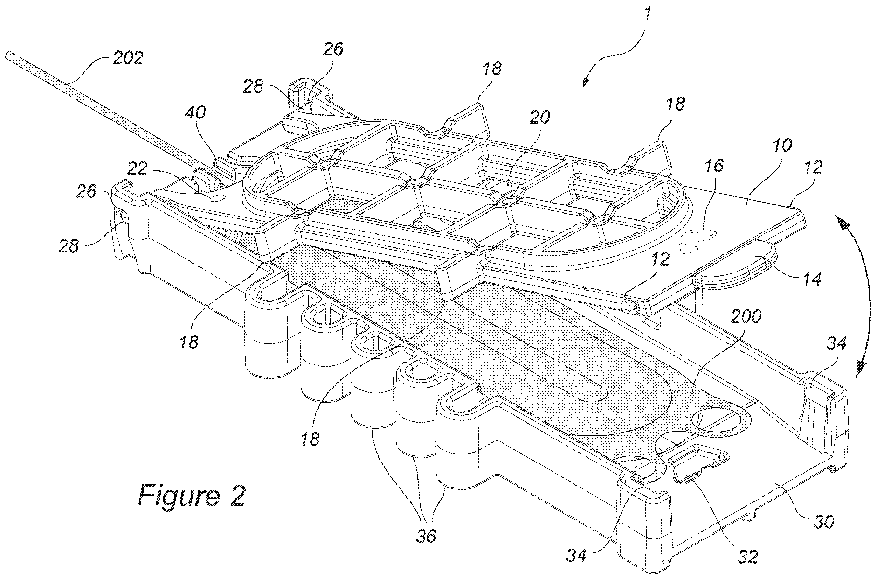

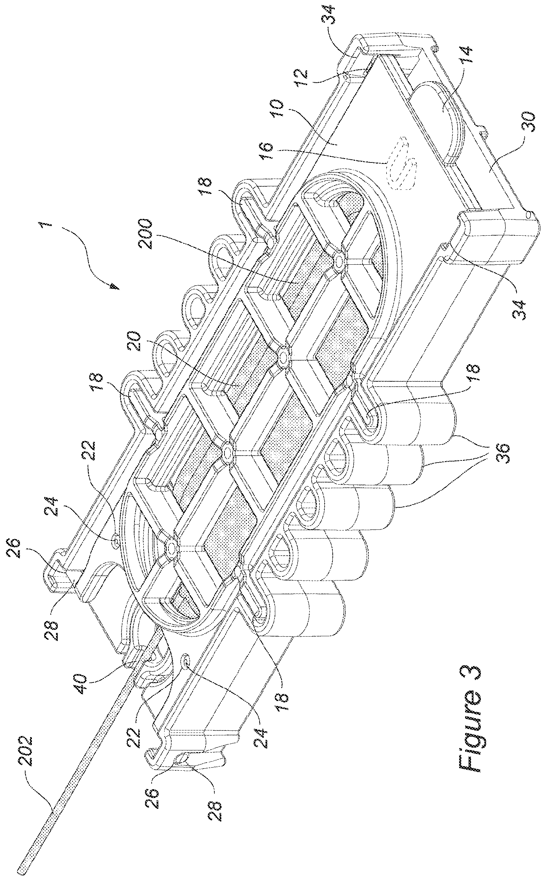

The case **1** may have any number of "coupling system,", "attachment system", "closing system" or equivalents thereof and refers to any system for closing, attaching, coupling or connecting one portion to another portion such as a hinge **26, 28,** snap **16, 32, 12, 34** button, string, hook, latch and loop fasteners or other types of fasteners used to couple the cover body **10** to the base body **30** and define a volume to receive materials, such as an implantable device and/or biologically active materials. **FIGS. 2-3** show the cover body **10** coupled to the base body **30** such that the cover body **10** moves between a first or closed position (FIG.3), wherein the implantable device **200** provided therein is partly concealed, and a second or open position (FIG.2), wherein the implantable device **200** provided therein is revealed or exposed. As illustrated, the cover body **10** and base body **30** can be coupled by one or more, such as two, coupling system to secure the cover body **10** and base body **30** together in a manner which allows the cover body **10** and base body **30** to move independently of the other. One such coupling system is illustrated in **FIG. 1****,** for example, a hinge mechanism **26, 28,** that pivotally couples the cover body **10** to the base body **30** relative to each other. The hinge mechanism **26, 28** generally comprises a pivot shaft or rod **28** inserted into an aperture or bore **26.** As illustrated the rod **28** is attached to the distal end of the cover body **10,** but it can also be attached to the base body **30.** The pivot rod **28** may be integrally formed with the cover body **10** or the base body **30,** or it may be provided as a separated component. Another way to attach the cover body **10** and base body **30** to each other is a latching system **16, 32, 12, 34** as illustrated in **FIG. 1****.** Such a system can be provided to lock the cover body **10** and the base body **30** at the most proximal end **16, 32,** or on the side walls **12, 34.** One purpose of the latching systems shown herein is to prevent the implantable device **200** from sliding out of the case **1** or from moving or traveling while housed in the case **1.** As illustrated, a user would use a snap **14** to apply a deliberate force against a friction fit of the snap and open or uncouple the latching system **16, 32, 12, 34** before loading or retrieving the implantable device **200** inside the case **1.** Although one side wall latching system **12, 34** is shown, other similar latching system can be incorporated throughout the long axis of the case accordingly and will depend, in part, on the shape and design of the implantable device, instrument, component, apparatus, element, material and the like encased therein.

In another aspect, the case consists of at least one registration hole **22** located generally on the cover body **10** and its complementary matching pin **24** on the base body **30,** such that the pin **24** inserts into the registration hole **22** therein as shown in **FIG.1** and provides a feature for interacting or connecting with the implant tool, such as a deployer, an embodiment of the invention as described in more detail below. Another coupling system is any number of feet projections **18** on the cover body **10,** which in **FIGS. 1-4** are shown to fit inside the internal aspect of a side walls of the cover body and base body, or finger grip **36.** For example, FIG.1 depicts one embodiment whereby the feet projections **18** fit or connect or slot into one section of the finger grip 36. Such feet projections are useful for at least preventing movement or travel of the cover body **10** relative to the base body, prevent the collapse of the cover body onto the base body, and holds or secures the implantable device **200** therein.

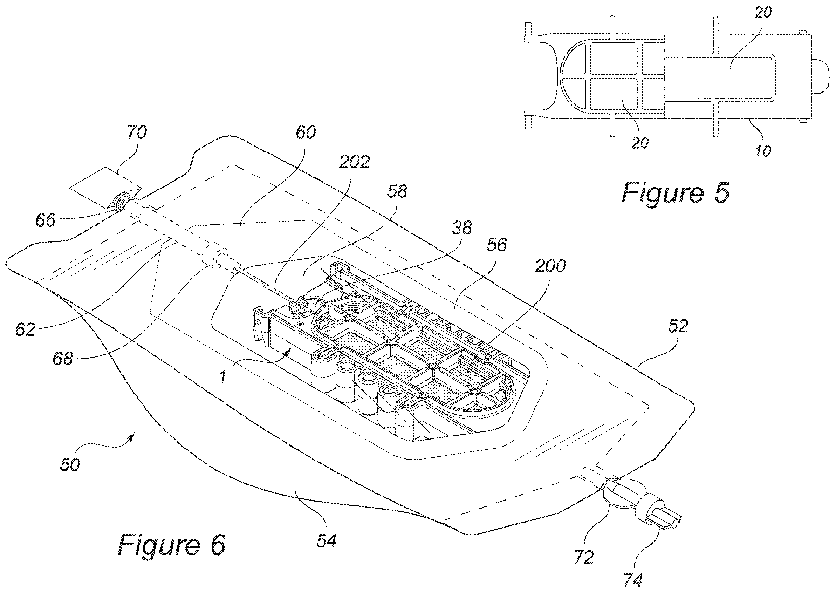

**FIGS. 1-4** show the cover body **10** and base body **30** consisting of at least one opening or window **20.** The windows **20** are configured to allow passage of the one or more biologically active materials between the interior and the exterior of the implantable device **200** therethrough. Although as illustrated the windows are of rectangular or semi-circle in shape, the windows **20** can be of any shape, design or number, which will in part depend on the shape and design of the device, instrument, component, apparatus, element, material and the like encased therein. **FIGS. 1-4** show the window **20** configurations on the cover body **10** and the base body **30** as being symmetrical, but they need not be symmetrical so long as they allow movement of one or more biologically active materials into and out of the implantable device. Alternatively, **FIG. 5** shows a window **20** configuration whereby the cover body **10** includes one large rectangular shape window **20,** while the base body **30** includes more than one window **20** type. Still depending on the type and manner of device, instrument, component, apparatus, element, material and the like encased therein, the windows may further include a mesh or thin polymer, film or matrix, which certain of these polymers or films can allow for the visualization of the implantable device and provides similar permeability and passage of nutrient medium.

The implantable device **200** illustrated herein is a macro-encapsulation implantable device further consisting of welded semi-permeable polymer materials and at least one implantable device port **202** which is used to fill or load a therapeutic agent into the implantable device **200.** However, modifications that do not depart from the device case embodiments described herein can be used to hold various implantable devices. And, in fact, Applicants have described various planar and non-planar (e.g. 3-dimensional) implantable devices that are contemplated including but not limited to self-expanding implantable devices, large capacity or macro-encapsulation, planar and non-planar implantable devices, or 3-dimensional macro-encapsulation implantable devices. Other encapsulation implantable devices have been described by Applicant, for example, PCT/US2014/022109, 3-DIMENSIONAL LARGE CAPACITY CELL ENCAPSULATION DEVICE, filed March 7, 2014; and U.S. Design Application Numbers: 29/408,366 filed December 12, 2011; 29/408,368 filed December 12, 2011; 29/423,365 filed May 31, 2012; 29/447,944 filed March 13, 2013; 29/484,363, 29/484,359, 29/484,360, 29/484,357;29/484,356, 29/484,355, 29/484,362 and 29/484,35, titled 3-DIMENSIONAL LARGE CAPACITY CELL ENCAPSULATION DEVICE and filed March 7, 2014.

**FIGS. 1-3** show the case **1** having a port sealing stage area **38,** which includes a cut-out or opening in the cover body **10** and base body **30** that allows for at least welding or any sealing instrument that is capable of welding and/or sealing an implantable device port **202** once the implantable device **200** is filled. The welding or sealing instrument can be hand held or mounted. Alternatively, the case 1 may also have a port guide **40** to align the port **202** in the port sealing stage area **38.**

To maintain the integrity of the implantable device **200,** the case **1** may generally also include side walls having rigid or not rigid grips, for example finger grips **36,** which allow for easy handling and manipulation of the case **1** as shown in **FIGS. 1-4****.** This is beneficial, e.g. during the device cell filling process and/or retrieval and transfer of the device, or manipulation by the handler. **FIGS. 1-4** show five finger grips **36** on each side wall of the case **1,** however, **FIG. 5** shows other nonlimiting, nonexclusive finger grip configurations including indented fingers grips **120, 122,** textured finger grips **124, 126, 128,** engraved or etched finger grips **124,** and rubberized or textured sticky finger grips **124, 128.** Techniques and methods for making finger grips are well known in the art. Alternatively, should handling, assembling, filling, sealing the implantable device port, transferring, storing and shipping, and deploying the implantable device become automated; the finger grips can be optimized for such automation or not be incorporated, or incorporated elsewhere in the case.

In one embodiment, the implantable device **200** can undergo various quality controls testing while inside the case **1.** Depending on the quality control testing, various one testing parameters will be used. For example, a dry or wet visual inspection using the naked eye and/or a microscope can determine the quality of the implantable device. In one embodiment, a dry visual inspection of the implantable device **200** can be performed first outside of the device case **1** followed by inside the case **1** using microscopy. In one embodiment, a wet visual inspection can also be performed by submerging the implantable device in a petri dish filled with isopropyl alcohol (IPA) and the implantable device **200** is inflated for about 30, 40, 60, 80, or 100 seconds during which period visual inspection for both sides of the implantable device is performed. For both dry and wet visual inspections, the quality value index could be a breach (e.g. a tear, a perforation, a leak, or physical abnormalities that may lead to a potential breach and the like) of the device can be determined using the naked eye and/or a microscope.

In one embodiment, a pressure decay quality control testing can be performed on the implantable device **200** inside the case **1.** Pressure decay is a non-destructive test which will be performed on all implantable devices. In one embodiment, pressure decay testing can be preceded by dry and wet visual inspection of the implantable devices. Various instruments can be used for pressure decay testing including but not limited to the USON leak testers such as the Raptor, Sprint iQ, Qualitek mR, Optima vT and Vector; and preferably the USON Sprint iQ. For the pressure decay test, the device case **1** with the implantable device **200** therein is again fully submerged in a wetting solution containing IPA. The device should preferably remain still or unmoved during this testing as potentially any movement can affect the measurement. Pressure decay is a measure of decay over time. Implantable devices **200** that pass the pressure decay test will have quality index measurements or values of about 0.006 to about 0.020 psi for about 10, 20, 30, 40, 50, 60, 80, 100 seconds or more, preferably about 0.008 to about 0.010 psi for about 10, 20, 30, 40, 50, 60, 80, 100 seconds or more, and preferably for about 0.010 psi for about 10, 20, 30, 40, 50, 60, 80, 100 second or more.

In one embodiment, quality testing of implantable devices **200** in their respective case **1** protects the integrity of the device during quality control testing, reduces the number of manipulations or touches of the implantable device because it remains in the case **1** during quality control testing, sterilization, filling into the device fill pouch assembly as described in detail below, and filling of the implantable device **200** with therapeutic agents, e.g. pancreatic progenitor cells, PDX1-positive pancreatic endoderm cells, endocrine cells, and immature beta cells and the like.

The embodiments herein describe tools and instruments for use with or along with an implantable device having a therapeutic agent therein. Applicants are developing a cell therapy for diabetes, specifically an encapsulated cell therapy to treat diabetes, and have in described in detail various endoderm-lineage or definitive-endoderm lineage cells, specifically pancreatic-lineage cells for use with the embodiments described herein. For example, Applicants have described in detail mesendoderm and definitive endoderm-lineage type cells in at least U.S. Application Serial Nos. 12/099,759, entitled METHODS OF PRODUCING PANCREATIC HORMONES, filed April 8, 2008; 12/618,659, entitled ENCAPSULATION OF PANCREATIC LINEAGE CELLS DERIVED FROM HUMAN PLURIPOTENT STEM CELLS, filed November 13, 2009; 14/106,330, entitled IN VITRO DIFFERENTIATION OF PLURIPOTENT STEM CELLS TO PANCREATIC ENDODERM CELLS (PEC) AND IMMATURE BETA CELLS, filed December 12, 2013; 14/201,630, filed March 7, 2014; and PCT/US2014/026529, IN VITRO DIFFERENTIATION OF PLURIPOTENT STEM CELLS TO PANCREATIC ENDODERM CELLS (PEC) AND ENDOCRINE CELLS, filed March 13, 2014. In one preferred embodiment, the implantable device consists of a therapeutic agent, a living cell, an endoderm-lineage cell, a definitive endoderm-lineage cell, a progenitor cell, a progenitor cell differentiated from stem cells (or a human embryonic stem cells including those derived from methods now known or to be discovered in the future, fetal stem cells, cord blood stem cell, induced pluripotent stem cells, reprogrammed cells, parthenote cells, gonadal germ cells, and mesenchymal, or hematopoietic stem cells, a pancreatic progenitor cell), a PDX-1 positive pancreatic progenitor cell, an endocrine precursor cell, an endocrine cell, an immature beta cell, an immature islet cell,

### Device Fill Pouch Assembly (DFPA)

**FIG. 6** illustrates a nonlimiting, nonexclusive embodiment of a device fill pouch assembly (DFPA) **50** that functions both as a sterilization bag for the sterilization of the case 1 and implantable device **200** therein, and an assembly to facilitate filling of the implantable device, e.g. cell filling of the implantable device.

As illustrated in **FIG. 6****,** the DFPA **50** is an assembly or system consisting of double peel pouch formed by peelably adhering a first sheet **52** and a second sheet **54,** the first sheet **52** having a clear, peelable transparent window **58** for viewing the implantable device **200** and case **1** therein and an inlet and outlet system. The transparent window **58** consists of frangible (pull-away) seals along the periphery **56, 60.** The inlet system can consist of a spacer tube **62,** which can be permanently affixed and fitted to any fluidic fitting including but not limited to microbore Luers **66, 68,** or detachably attached the any fluidic fitting. In one embodiment, the spacer tube **62** can receive the implantable device port **202** therein hence the port **202** is inserted into the spacer tube **62** there through can be further connected to a cell reservoir, for example, which cell fills the implantable device therein. The outlet system consists of drain port **72** and a drain port cap **74** for draining or depleting or emptying excess media, e.g. excess nutritive media. The most exterior parts of the inlet and outlet system may further comprise a cap **74** e.g., a swabable cap, and one or both systems covered in a shroud or sheath **70.** Also, it is understood that these inlet and outlet system can be modified or optimized to fit the purpose of the cell or therapeutic agent intended for loading into the implantable device. In one embodiment, suspension cell aggregates are loaded into the implantable device, and certain microbore features help minimize, reduce and prevent clogging or congestion during the cell filling process. The DFPA is wholly sterilizable or made from a sterilizable material or paper, alone or preferably with the case **1** and implantable device **200** therein. In this instance, the case **1** and implantable device **200** are sterilized at the same time and no further separate assembly of the case and implantable device is necessary post sterilization.

The DFPA **50** also functions as an assembly or system, specifically, a container or reservoir, for filling the implantable device **200** with living cells or other therapeutic agent(s). In the instance of a cell therapy for treatment of diabetes mellitus, the implantable device **200** is filled with living pancreatic progenitor cells, e.g. PDX1-positive pancreatic endoderm cells, or PEC, or endocrine precursor cells, or endocrine cells, or immature beta cells. Methods for filling an implantable device including U.S. Patent No. 5,964,261 to Baxter International Inc. ("'261 Baxter patent") which describes manually wetting, sterilization, filling via a syringe, and sealing of an implant devices. However, in contrast to the implantable devices described in the '261 Baxter patent, the implantable devices envisaged herein have at least 6-fold the volume capacity as that of the much smaller volume Baxter implantable devices, i.e. 250 µL versus 4.5 µL, 20 µL and 40 µL; and as such, the instant invention contemplates a scaled manufacturing and processes for wetting, sterilization, filling, and sealing of much larger capacity implantable devices. For example, these other implantable devices need to be pre-wetted or prepared before the cells are loaded by injecting or bathing the implantable device in a liquid mixture adequate to pre-wet it, e.g., sterilized water, saline or about 70% to 100% ethanol into DFPA **50** bag through the inlet system. In one embodiment herein, the interior of the implantable device can be primed or wetted, e.g. with cell culture media (not ethanol) before immediately loading the cell aggregate suspension into the implantable device. The priming solution can in this instance be the same solution (or culture media) as that the cell aggregates are suspended in. This is at least one less step than in previously disclosed methods including the '262 Baxter patent and residual ethanol is not a concern. The solution (cell culture media, other nutritive media or other liquid mixture) is drained out of the bag through the outlet system taking steps to remove any air from the implantable device. If water or ethanol is used to pre-wet the implantable device, then saline or cell culture media can be used to flush any residual water or ethanol from the device. If pre-wetting the implantable device is required for cell loading, care must be used to make sure that air bubbles do not get trapped in the fluidic path (e.g. the inlet or outlet systems), which may interference with cell loading. The volumes of cell suspension and media amounts drawn up into the syringe will depend on the maximal volume capacity or size of the implantable device. If desired, less dense cell concentrations may be used. For example, 1 x 10⁷ cells in a final volume of 30 µL may be used for the 20 µL implantable device. The pre-wetting and filling methods described in the '261 Baxter patent consist of many parts and intended for research and not clinical use, and therefore difficult to maintain a sterile and / or aseptic environment necessary for regulatory compliance for any cell product. Because the DFPA 50 can have a dual function (sterilization and filling assembly), it reduces the likelihood that the implantable device inside the case will be compromised, breached or contaminated. Further, because the '261 Baxter patent devices are significantly smaller in size and volume capacity than the devices in the embodiments herein, cell loading by way of a syringe is feasible although not practical for scale up purposes.

Since sterilization may be conventionally performed with radiation (e.g. ionizing radiation, gamma irradiation, electron beam irradiation), dry heat exposure or high-temperature moisture (e.g., steam (wet heat) exposure, chemical (e.g. ethylene oxide exposure and the like), materials used for the DFPA **50** of the present invention are of a material that allows the effective elements (gases) and water vapor to pass through while not allowing germs or liquid water to pass through. A sterilization systems that is capable of simultaneously sterilizing the DFPA **50,** the case **1** and the implantable device **200** is preferred, however, certain case **1** or implantable device **200** materials may not be compatible with high temperatures nor irradiation, and ethylene oxide exposure is a reasonable sterilization approach. Also, sterilization approaches may also dictate choice of sterilization package materials. The packaging materials described herein are compatible with most sterilization methods contemplated above.

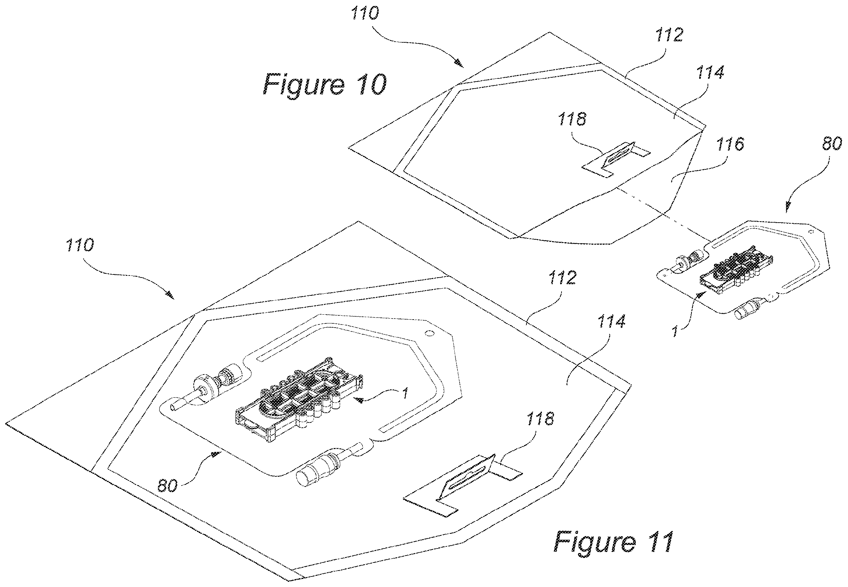

The DFPA **50,** in particular the sheets **52, 54** of the illustrated embodiment, is formed by peelably adhering base materials or sterilization paper, preferably made of polyester, polyvinyl chloride, polyethylene, polypropylene, ethylene copolymers, ionomer resins or material such as a gas-permeable polyethylene or polypropylene non-woven fabric, strong, lightweight, flexible, resistant to water, chemicals and abrasion. Alternatively, a thermoplastic transparent resin film that exhibits a proper degree of adhesive strength and that serves as a seal layer between the first sheet **52** and second sheet **54** can be laminated between the sheets without harming the gas permeability of the sheets, and the both materials can be sealed and adhered to each other. In other embodiments, the DFPA **50** consists essentially of the same properties as that described in detail for the shipping bag **110** in figure **FIG. 11** below.

The transparent window **58** is preferably made of a biaxially-oriented polyethylene terephthalate (trade name is Mylar) or polyester film made from stretched polyethylene terephthalate (PET) and is used for its high tensile strength, chemical and dimensional stability, transparency, reflectivity, and gas and aroma barrier properties. However, other materials with substantially similar properties as that indicated above for each of the base materials for the DFPA **50** or the transparent window **58** are envisaged by the present invention, including any clear polymer film which maintains a sterile barrier under normal handling would be acceptable (e.g. polyethylene).

### Device Case Storage Bag

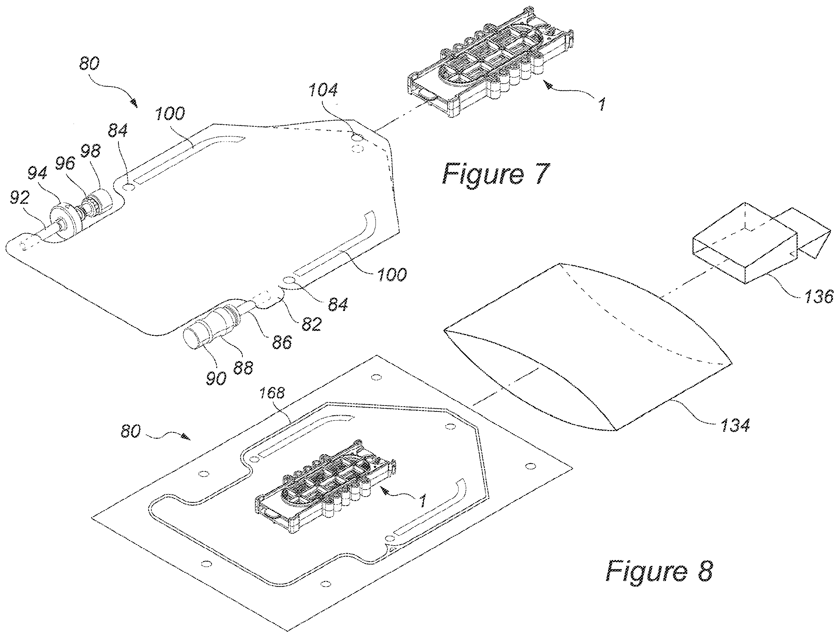

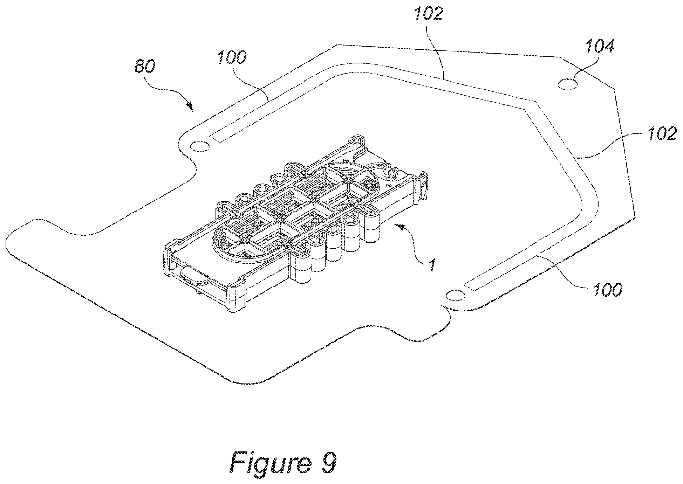

**FIGS. 7-9** illustrate a nonlimiting, nonexclusive embodiment of a container or storage bag **80** useful for storing an implantable device **200** filled with cells. **FIG.9** is one perspective view of a bag **80** made in accordance with this invention. The storage bag **80** may be made of conventional construction, including a pair of plastic sheets sealed at periphery **168** and side and upper frangible seals **100,** a hanger provision **104** for maintaining the storage bag **80** in an upright position if necessary and various registration holes **84.** Alternatively, as illustrated in **FIGS. 7-8****,** the storage bag **80** may also include access ports **86, 92** permanently (e.g., UV bonding) or detachably connected to fluidic fittings **88, 94, 96** and caps **98, 90.**

In accordance with this invention, the storage bag **80** is made from a transparent, flexible, sterilizable flexible and strong plastic including but not limited to polyethylene and Ethylene-VinylAcetate (EVA), polyolefins, polyolefin mixtures, polycarbonate, polysulfone, polystyrene, polyvinyl chloride (PVC) plasticized with plasticizers known in the art as di-2-ethylhexyl phthalate (DEHP or DOP) or, in some cases certain triesters of trimellitic acid such as tri-2-ethylhexyl trimellitate (TOTM or TEHTM); and preferably polyethylene and EVA. The material selected for the storage bag **80** is a coextrusion of polyethylene and EVA, which is advantageous over standard PVC materials because it reduces or prevents plastics leaching into the lumen of the storage bag **80** where the cells are encapsulated, and it reduces general environmental mercury levels typically used in traditional PVC manufacturing. These films and the above PVC plasticizers are described more fully, for example, in U.S. Patent No. 4,280,497 to W. Warner et al. and U.S. Patent No. 4,222,379 to D. Smith. However, similar plasticized bags have been found to yield a detectable amount of the ester type plasticizer into the plasma of the blood as it is stored in the bag for a period of days. Accordingly, one embodiment of a storage bag of the invention does not preferably leach plasticizer into the interior of the storage bag, which holds and stores a living cells encapsulated inside an implantable device **200** and case **1** therein. Moreover, the cells encapsulated in the implantable device require an exchange of gases and nutrients, so any flexible plastic material should preferably be gas permeable or impermeable so as to meet the metabolic requirements of the cells stored therein; particularly for long-term storage, to help keep the cells viable.

**FIG. 8** shows alternative components for loading the device case **1** into the storage bag **80** to help maintain an aseptic environment and thereby minimally contacting or touching the case **1** and/or implantable device **200.** As shown, there is a first sleeve **134** for protecting the upper or top or proximal portion of the storage bag **80** during subsequent sealing for example, and a second sleeve or guide **136** insertably inside the first sleeve **134** to slide the case **1** and filled implantable device **200** into the storage bag **80.** After the case **1** and filled implantable device **200** are placed into the storage bag **80,** the bag is filled with a cell culture storage medium including but not limited to base mediums such as Dulbecco's Modified Eagle's Medium (DMEM), or CMRL media developed by Connaught Medical Research Laboratories (CMRL), StemPro hESC SFM (Life Technologies), or any appropriate storage medium such that the medium bathes and covers completely and keeps wet the implantable device **200** therein. Once the storage bag **80** is filled, the upper opening is then sealed and can be continuous with the side frangible seal **100** to form a complete seal of the device case, implantable device and storage medium therein. Storage of the filled implantable device **200** in such a manner is suitable for up to 1 day, 2 days, 3 days, 4 days, 5 days, 6 days, 7 days, 8 days, 9 days, 10 days, 11 days, 12 days, 13 days, 14 days, 15 days, 16 days, 17 days, 18 days, 19 days, 20 days, 25 days and 30 days or more at an appropriate storage temperature to maintain viability of the living cells before transplant.

In one embodiment, the storage bag **80** is formed by creating a wholly peripheral seal **168** and a frangible seal **100,** then cutting out the bag such that bottom and side sections of the periphery seal **168** are retained, while the top side and top sections of the periphery seal are not (e.g. these sections are cut out or left off); but the frangible seals **100** remain in-tact. Alternatively, the periphery seal **168** can remain intact alongside the frangible seal **100** depending on the use, e.g. if the use does not require use or peeling away of the frangible seal **100,** then the periphery seal **168** can be retained except for the top opening.

### The Shipping Bag

**FIGS. 10-11** illustrates a non-limiting, nonexclusive shipping bag **110** in accordance with the present invention. The shipping bag **110** is formed from first and second sheets **114, 116** positioned in opposing face-to-face relation and sealed to one another along three sides **112** to form a pouch, leaving an opening **116** along the remaining side suitable for receiving articles, for example, a device case storage bag **80.** In the embodiment shown, the sheets **114, 116** are generally square or rectangular in shape and they are sealed to one another along seal areas **112** adjacent opposite side edges of the bag, and along an angularly extending seal area on one side edge of the bag. After the shipping bag **110** has been filled with its contents by the user, it may be sealed aseptically to close the opening keeping the contents inside sterile. Sealing of the sheets **114, 116** to one another may be carried out using conventional heat or ultrasonic sealing equipment as is well known in the art. To maintain aseptic handling, the shipping bag **110** may further comprise one or more handle(s) **118** attached to the exterior of the bag for use in opening and keeping it opened without touching the interior of the bag while the contents are being filled.

In one embodiment, the shipping bag material of **FIG. 11** is substantially similar in properties to the DFPA **50** of **FIG. 6****.** Both consist of a heat sealable transparent thermoplastic polymer film inner layer and forms the laminate between two sheets typically made from a sterilizable material or paper. Preferably, the sheet is a polyolefin material, suitable examples of which include polypropylene, polyethylene, ethylene copolymers such as EAA, EMA, EVA and ionomer resins such as Surlyn from DuPont or Iotek from ExxonMobil. The laminate and sheets may suitably have a thickness of from about 0.5 mils to about 4.0 mils, more preferably about 1.5 to about 3.0 mils, and most preferably about 2 mils. Alternatively, as illustrated in **FIG. 6** for the DFPA **50,** the sheets **114, 116** may consist of a transparent material capable of imparting strength, puncture resistance, dimensional stability and durability to the bag. Suitable materials for the transparent material include polyethylene terephthalate (PET), nylon, polypropylene, polyethylene and cellophane. Particularly preferred are biaxially oriented films such as biaxially oriented PET and biaxially oriented nylon. The sheets may have a thickness of from about 0.36 to 2.0 mils, more preferably from about 0.48 to 1.0 mils, and most preferably about 0.48 mils (48 ga). The sheets **114, 116,** transparent or not, may additionally have a moisture barrier, for example, a molecularly oriented polychlorotrifluoroethylene (PCTFE) fluoropolymer film. The PCTFE film is transparent, biochemically inert, chemical resistant and free from plasticizers and stabilizers. Preferably the molecularly oriented PCTFE film is a monoaxially oriented film. PCTFE fluoropolymer films are sold by Honeywell, Inc. under the Aclar registered trademark.

Conventional lamination and sealing methods include but are not limited to using a spray adhesive, roll coating, knife over roll coating, wire rod coating, or gravure coating. Suitable adhesives include solvent based, water based or solventless adhesives including acrylic adhesives, epoxy cured polyester urethanes, moisture cured polyester urethanes and isocyanate terminated polyester adhesives. Alternatively, the inner (or middle) laminate layer can be formed directly on the sheets **114, 116** by extrusion coating; and if there is the transparent outer layer, it can be laminated directly to the sheets **114, 116** using known adhesives and techniques as described above. If desired, the inner surface of the transparent layer may be reverse printed prior to laminating to provide a layer of printing with product label or graphics or other information. The sheets **114, 116** may also be surface printed prior to or post lamination.

### The Sizer

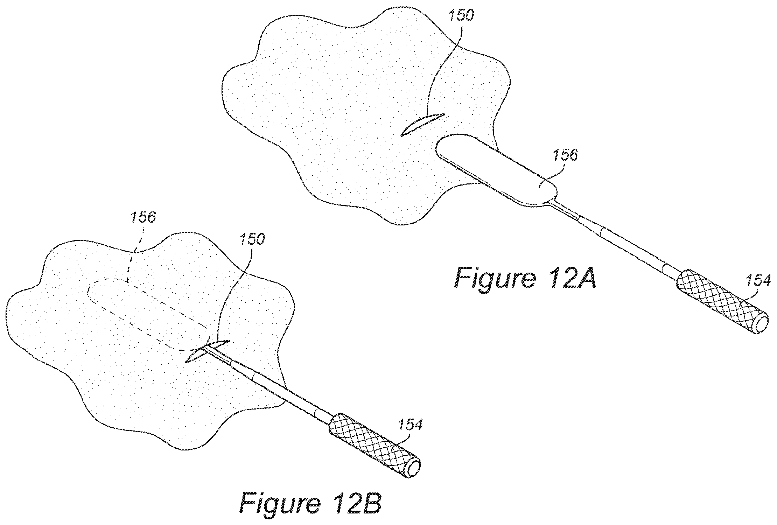

**FIG. 12** illustrates a nonlimiting, nonexclusive surgical sizer in accordance with the invention. The sizer **150** includes a distal or pocket-forming portion **156** and proximal or handle portion **154.** In the example shown, the pocket-forming portion **156** is approximately the shape, or is approximately the shape that sizes the cavity, of the implantable device **200** to be implanted. Certain detailed aspects of the actual implantable device, such as its port and surface details need not be reproduced in the pocket-forming portion **156;** and in fact, omitting such details may serve to simplify manufacturing as well as reduce the number of features that could potentially damage the tissue if not properly fabricated and create a cavity that allows implantable device **200** to conform to the target implant site, regardless of overall geometry. For example, in one embodiment, the sizer is slightly tapered with a sharper leading edge useful to expand out the tissue at the implant site. In one embodiment the pocket-forming portion **156** can have non-planar geometries, for example, the precise shape and curvature of the pocket-forming portion **156** may be bent or tailored to the particular surgical procedure, anatomical site, and access route. Further, the pocket-forming portion **156** may be slightly larger than the actual dimension of the implantable device, for example, it may be scaled to 105%, 110%, 115%, 120%, 125% or 130% or more of the dimension of the implantable device. The pocket-forming portion **156,** like the implantable device **200** it replicates, may be shaped to follow the contours of the relevant anatomical implant site so as to prevent causing damage to delicate tissue, for example contoured to the curvature of the back, arm, abdomen, flank, leg, etc.

The proximal or handle portion **154** is an elongated substantially planar handle portion with a grippable handle **154.** The handle **154** may be etched, engraved or textured, contain a cut-out (or groove) that is anatomically compatible with a human thumb or index and middle finger to provide the surgeon with an appropriate gripping surface. The cut-out may take the form of a circular hole, an elongated slot, or other shape (e.g., a hook-shape), and have a textured grip for additional feedback. Alternatively, the entire proximal or handle portion including the elongated shaft and the grippable handle **156** can be textured, while keeping the distal or pocket-forming portion **154** relatively smooth. In still another embodiment, the pocket-forming portion **156** may be permanently or detachably coupled to the proximal or handle portion **154.**

Still, the sizer **150** can have non-planar geometries, for example, the precise shape and curvature of the proximal or handle portion may be bent or tailored to the particular surgical procedure, anatomical site, and access route. For example, the handle portion may have different sections with different radii of curvature (including planar sections and curved sections). So, depending on the particular application, a sizer **150** in accordance herewith may include differently curved ergonomic handle portion to aid the surgeon in placing the implantable device into the applicable anatomic region, e.g., a particular layer of the dermis, or between the muscle and the dermis.

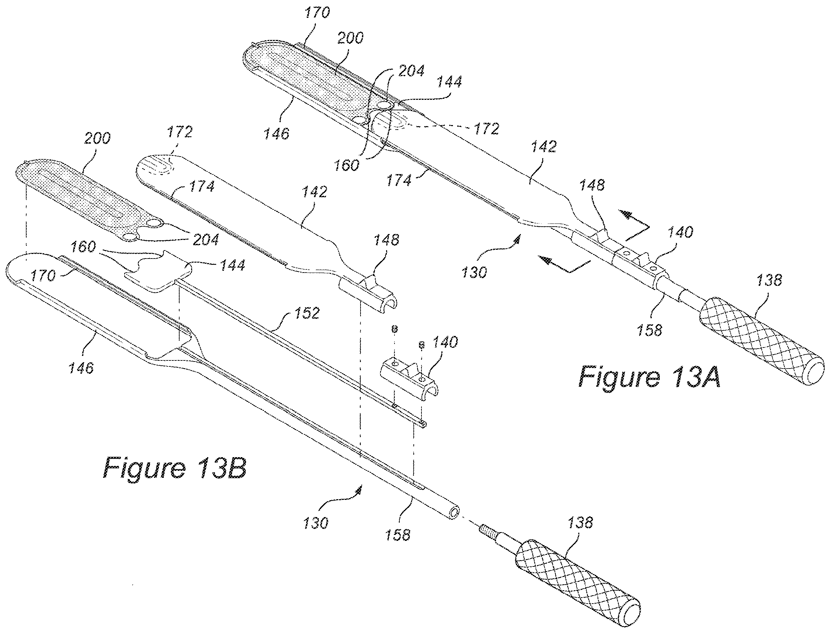

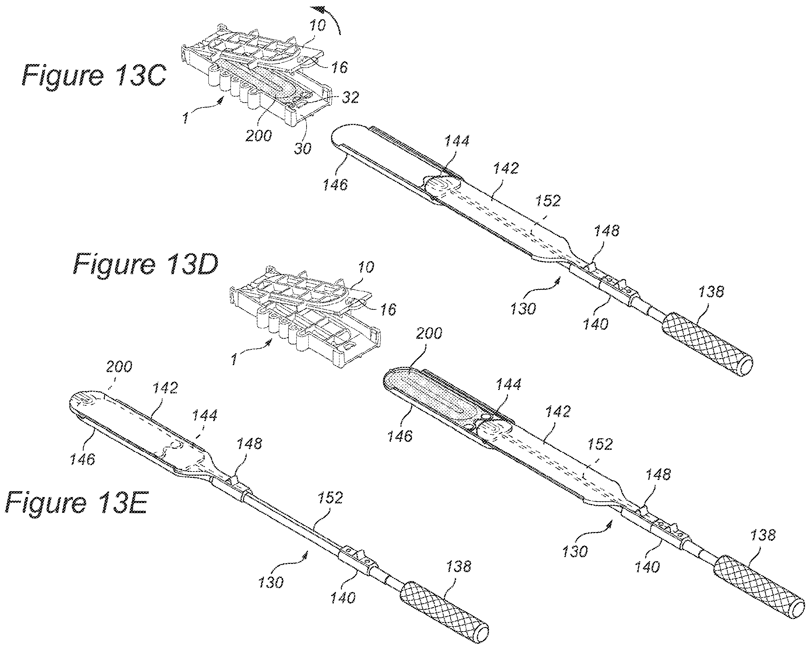

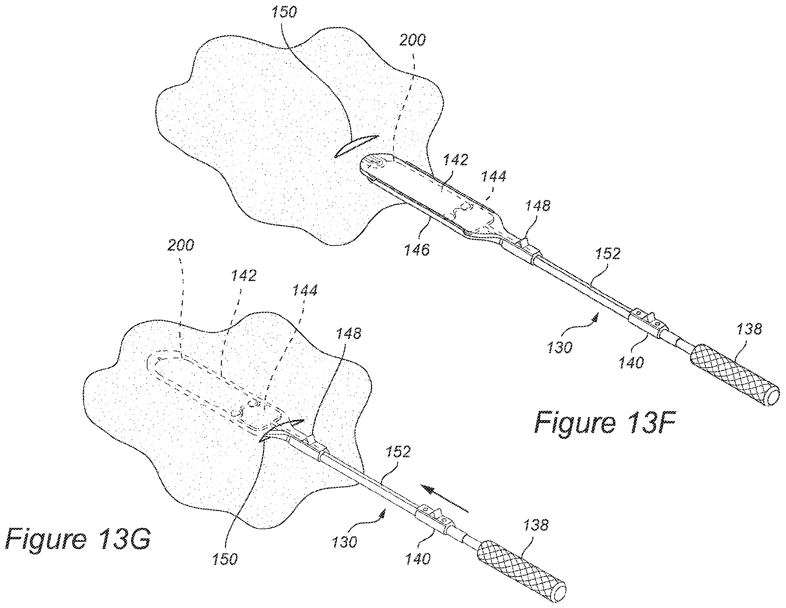

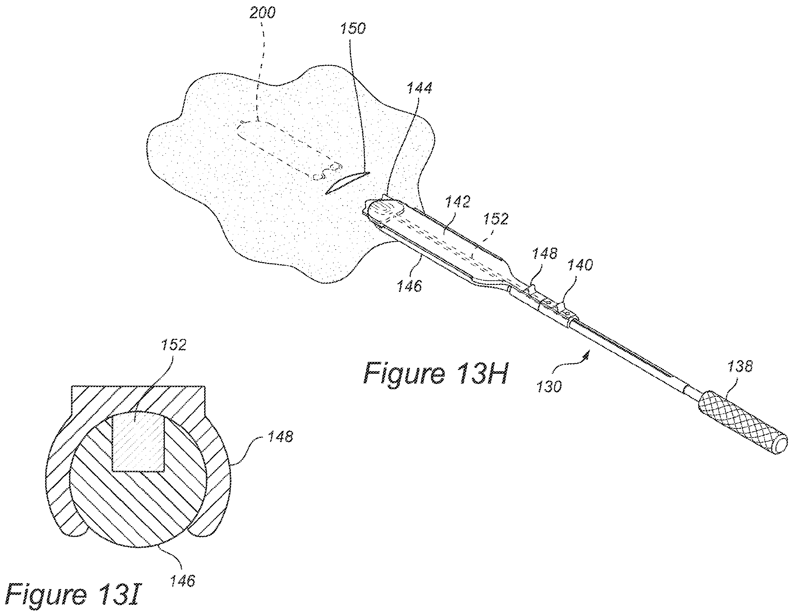

Also, the sizer**150** may have various markings to assist with use. For example, the pocket-sizing portion **156** may contain markings, which may correspond to the location of anchoring features, or other relevant structural features of the implantable device, or may act like a ruler to measure an implant site, e.g., the pocket-sizing portion **156** has a mark to indicate the placement of anchoring sutures corresponding to suture rings **(204** in **FIG. 13****)** in one embodiment of the implantable device **200.** The proximal or handle portion may also have markings on the elongated shaft and/or handle, which may aid the surgeon in realizing when the pocket-sizing portion should not be pushed any farther in the posterior direction for example.

Also the sizer may comprise a marking tool such as a felt-tip marker, cauterizing tool, or other comparable marking tool to identify certain locations proximate the anatomic location where an implant should be placed.

Alternatively, the distance markings on the distal and proximal portions may have associated holes or slots that allow the distance to be marked on the tissue using any one of the methods mentioned above. In one embodiment, the pocket-sizing portion **156** may include a hole marking, which allows a pen or cauterizing tool, for example, to mark the desired placement of the implantable device, or to mark the boundaries of the implantable device, or to mark the placement of any sutures. Optionally, additional depth markings at specific distances (e.g., in millimeter increments indicative of the distance to the far edge of the pocket-forming portion) along the handle portion may provide a guide to let the surgeon know when the implantable device has reached its optimum or required depth. The markings may, generally, be tactile and/or visual in nature; for example, they may be grooves or notches perceptible by touch, or simply lines drawn onto the handle portion. Of course, the types and locations of markings described herein are exemplary only and those of skill in the art will readily be able to adapt the markings to surgical tools for a variety of other applications.

In some embodiments, sizing and marking functionalities are not integrated into the same instrument, but instead are provided by two separate surgical instruments.

Materials for manufacturing of such a sizer and /or marking tool are described in detail below.

### The deployer device

**FIG. 13** illustrates a nonlimiting, nonexclusive embodiment of a deployment device ("deployer", or also referred to as a deployer device, deployer tool, deployment tool, delivery tool, delivery device, implantation tool, or implantation device) of the present invention. As used herein a deployer or its equivalent such as "apparatus", "assembly", or "planar assembly" or "system" refer to a tool or instrument capable of retracting, holding, transferring, delivery and deploying a device, such as an implantable device, therein. Generally, the deployer **130** includes at least a proximal and distal end or portions. The distal end also functions as the holder or deployer end **142, 146, 144** and the proximal end also serves as the handle end **138.** The total length of the deployer is preferably longer than a length of a deployable implantable device.

The distal end further including at least two holder plates **142, 146,** a middle moveable deployer plate or element **144,** an elongate shaft **130** the proximal end of which includes a handle **138.** The holder plates comprise a cover plate **142** (or cover) and base plate **146** (or base) (or first and second holder, or a first and second plate, respectively), such that the base plate is affixed to a shaft **158** and detachably attached to a handle **138,** for example a grippable handle. The base plate **146** may have side walls **174,** rails or guides which help placement as well as hold the implantable device in place and prevent the implantable device from slipping. Similarly, the cover plate **142** may have rails or guides **170** which assists in the movements of the cover plate, deployer and base plate relative to each other. When assembled the cover plate **142** and base plate **146** together form a holder for holding the implantable device **200.** The distal end further includes a movable deployer plate **144** in between the cover plate **142** and base plate **146** of the holder. The cover plate **142** and deployer plate **144** are assembled and detachably attached or connected to the base plate **146** by way of the base shaft **130** which consists of a rod-shaped shaft **158** with a superior groove along the axis of the shaft **158.** The cover plate **142** may also be detachably attached or connected to the base plate **146** by means of a seat, channel, or groove along the inside wall of the base plate **146.** The deployer plate **144** is connected or affixed to a deployer shaft **152,** which is smaller in diameter than the base holder plate shaft **158,** and thereby capable of inserting into the groove or cut out of the base shaft **158** such that it is capable of moving axially along the base shaft **158,** or specifically of moving along the top or superior portion of the base shaft **158.**

The deployer plate **144** and the cover plate **142** further include control elements **140, 148** on or affixed to the cover plate **142** and the deployer shaft **152,** respectively, with the control element **148** (also referred to as the first control element, cover plate control element or cover control element) for the cover plate **142** affixed distal to the deployer control element **140** (also referred to as the second control element, deployer plate control element or deployer control element). The deployer plate **144,** the deployer shaft **158** and deployer control element **140** are collectively referred to as the deployer mechanism or deployer apparatus. The deployer plate may also be modified or optimized, e.g. towards the distal end, to accommodate various types of implantable devices. For example, FIG.13B illustrates an exploded view of the device with a deployer plate, whereby the distal end **160** resembles that of the suture holes or ears **204** of the implantable device.

The interconnectedness of these parts is best illustrated in **FIG. 13I,** which shows how the deployer shaft **152** sits inside the groove of the base shaft **158** and whereby the control elements **140, 148** wrap around both, securing the deployer shaft **152** from leaving the base shaft groove. The cover plate **142** and deployer plate **144,** in particular the cover control element **148** and the deployer control element **140** can be threadably moveable inside the base plate **146** rod **158,** or ratchetably movable inside the same.

The control elements allow for independent axial movement of the cover plate **142** and deployer plate **144,** relative to the base plate **146.** The control elements **140, 148** can also be secured by lock tabs, thumb operable wheel, switch or snap or actuator by way of a rounded detent (e.g. a twist), additional force, or additional part and the like, which parts are available and known to one skilled the art. For example, as illustrated in **FIGS. 13C-E**, to cover and hold the implantable device **200,** the user unlatches the latching system (snaps open) the device case **1** and inserts the base plate **146** under the implantable device **200** by pushing (e.g. thumb control) and proximally (or back) the cover plate control element **148.** Since, the cover plate control element **148** is affixed distally to the deployer plate control element **140,** moving the cover plate control element **148** back towards the handle, or grippable handle **138,** also then moves the deployer plate **144** back as well. However, to entirely cover the implantable device **200,** the cover plate **142** can be moved distally (forward) to cover the implantable device **200** without while not moving or keeping in place the deployer plate **144** and deployer shaft **152.** See, for example, **FIG. 13D** whereby the two control elements **140, 148** are side by side or directly adjacent to each other, whereas in **FIG. 13E** they are not side by side but separated, preferably separated by approximately the same distance as length of the cover plate **142** and base plate **146** as well as the implantable device **200.**

The deployer **130** as illustrated herein is described for use with the described implantable device **200,** other planar and non-planar (e.g. 3-dimensional) implantable devices including semi-permeable planar and non-planar implantable devices are contemplated including but not limited to self-expanding implantable devices, large capacity planar and non-planar or 3-dimensional macro-encapsulation implantable devices. Other encapsulation implantable devices have been described by Applicant, for example, PCT/US2014/022109, 3-DIMENSIONAL LARGE CAPACITY CELL ENCAPSULATION DEVICE, filed March 7, 2014; and U.S. Design Application Numbers: 29/408,366 filed December 12, 2011; 29/408,368 filed December 12, 2011; 29/423,365 filed May 31, 2012; 29/447,944 filed March 13, 2013; 29/484,363, 29/484,359, 29/484,360, 29/484,357;29/484,356, 29/484,355, 29/484,362 and 29/484,35, titled 3-DIMENSIONAL LARGE CAPACITY CELL ENCAPSULATION DEVICE and filed March 7, 2014.

Once the implant site is prepared for implantation of the implantable device **200,** by way of the sizer **156** as described above, **FIGS. 13****-F-H** illustrate how the implantable device **200** is deployed (also referred to as a deployable implantable device). To deploy the implantable device **200,** the user with one hand pushes the deployer control element **140** distally (or forward) while with the other hand, at the same time, move the entire deployer **130** proximally (or backward), thereby deploying the implantable device **200** into the prepared implant site. The implant site can then be closed at the opening site **150.** Alternatively, the deployer plate **144** is held in place and the cover plate **142** and base plate **146** are pulled or moving away from the implantable device **200** and implantable site.

Although not illustrated, in one embodiment, the deployer device **130** may further include a grasping system at or near the distal or holder end, or as part of or attached to the deployer plate **144.** Such a grasping system is capable of detachably attaching to the implantable device **200** so that when the deployer plate **144** is retracted (moves proximally or backward), the implantable device **200** moves accordingly onto the base plate **146.** The grasping system may be controlled, however, from the proximal end of the deployer **130,** e.g. with a further control element or actuator similar to that shown for the other two control elements **140, 148** or just by the deployer control element **140.** In this instance, such a grasping system may grasp the implantable device suture holes or rings **204,** for example, using some type of hook.

Similar to the sizer **156,** the distal or holder or deployer end **142, 146, 144** can have non-planar geometries, for example, the precise shape and curvature of the distal or holder or deployer end **142, 146, 144** may be bent or tailored to the particular surgical procedure, anatomical site, and access route. Likewise, the sizer **156,** the deployer handle **138** may have non-planar geometries, for example, the precise shape and curvature of the handling portion may be tailored to the particular surgical procedure, anatomical site, and access route. The handle portion may have different sections with different radii of curvature (including planar sections and curved sections). So, depending on the particular application, a deployer **130** in accordance herewith may include differently curved ergonomic handling portions to aid the surgeon in placing the implantable device into the applicable anatomic region, e.g., a particular layer of the dermis, between the muscle and the dermis or other superficial tissue layers. In one embodiment, the rod or shaft **158** and handle **138** may have depth markings or an associated marking tool.

In the embodiments shown, like the sizer, the distal or holder end takes the approximate shape of the implantable device **200** to be implanted. Certain detailed aspects of the actual implantable device, such as its port and surface details need not be reproduced in the pocket-forming portion **156;** and in fact, omitting such details may serve to simplify manufacturing as well as reduce the number of features that could potentially damage the tissue if not properly fabricated. Still certain functional features of the implantable device **200** may be incorporated into the deployer to better retrieve, hold, transfer and deploy the implantable device, for example, the most distal end of the deployer plate **144** may adapt approximately the shape of the proximal end of the implantable device **200.** As illustrated in **FIG. 13B****,** the deployer plate **144** has two indentations which were adapted so that it matched the suture registration holes or rings **204** at the proximal end of the implantable device **200** in this instance. Other modifications can be made to accommodate other types of implantable devices. Further, the distal or holder end **142, 146** may be slightly larger than the actual dimension of the implantable device, for example, it may be scaled to 105%, 110%, 115%, 120%, 125% or 130% or more. Similar to the sizer **156,** holder portion **142, 146, 144** may be shaped to follow the contours of the relevant anatomical implant site so as to prevent causing damage to delicate tissue, for example contoured to the curvature of the back, arm, abdomen, flank, leg, etc.

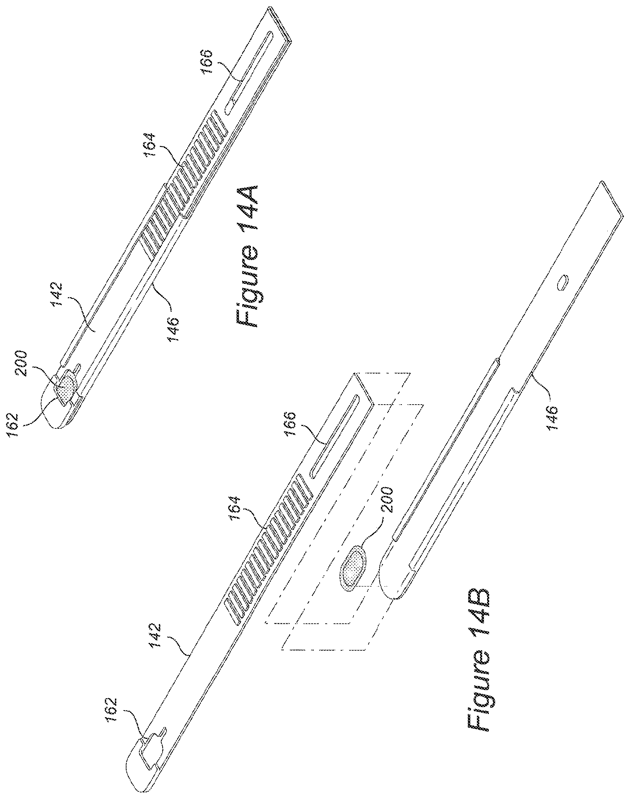

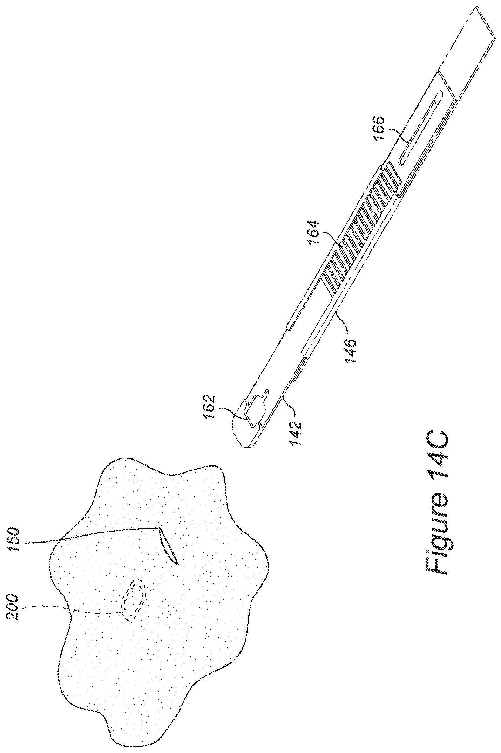

**FIGS.14A-C** illustrate another deployer embodiment of the invention. The deployer generally consists of an interconnected cover plate **142** and base plate **146,** without an intermediate deployer plate, operable control elements, or shaft handle as in the above embodiment. The cover plate **142** can alternatively consist of an opening or window **162** to view the implantable device **200.** In one embodiment, the attachment system between the cover plate **142** and base plate **146** are detachably attached by a notch on the base plate **146** that inserts into the groove **166** on the cover plate **142.** Movement of the two plates **142, 146** relative to each other can be accomplished by a texturing system or gripping system **164** to the cover plate **142,** for example, shallow ridges**164** as shown in **FIG.14****.** Still other embodiments similar to that described previously for the control elements **140, 148** (e.g. thumb operable switch or wheel, lock tabs or snaps by way of a rounded detent) of the deployer **130;** and that described for the finger grips **36** (indented grips **120, 122,** textured **124, 126, 128,** engraved or etched grips **124,** and rubberized or textured sticky grips **124, 128)** of the device case **1** can be employed or applied to the cover plate **142** herein. Delivering and deploying the implantable device **200** in such an embodiment does not depend on a deployer, rather moving cover plate forward, e.g. by pushing forward (or distally) on the ridges **164.**

In one embodiment of the invention, the deployer **130** may be equipped with any a number of holder system at the distal end, since the holder plates will be modified or adapted accordingly to the shape and design of the implantable device, while still keeping the same concept of a slidable system for transferring and deploying the implantable device.

In one embodiment of the invention, the deployer **130** may detachably attach or connect to devices, implantable or not, that are commercially available or currently exist and only nominal modification of the deployer as described here would be required.

In one embodiment of the invention, the control elements can take on other configurations and need not be thumb or finger activated elements as illustrated, for example, it can be a thumb operable wheel or a switch.

In one embodiment of the invention, the control elements may lock at various different positions along the length or axis of the deployer, such locking position may be dependent on the implantable device.

### Manufacturing of the device case, sizer and / or deployer

The device case, surgical sizer, marking tool, and deployer as described herein may be constructed using injection molding, machining, stereolithography, or other 3D manufacturing (e.g. 3D printing) procedures known to persons of skill in the art. The construction of these instruments may be adapted to its intended application and use. For example, a sizer, marker, or deployment tool intended for repeated use may be made of an autoclave-compatible material (i.e., a material that withstands the highpressure, high-temperature steam used in an autoclave to sterilize the tool), such as metal (e.g., stainless steel, gold, platinum, titanium, niobium, nickel, nickel titanium, colbalt-chrome alloys, molybdenum or molybdenum alloys, or an alloy such as nitonol (a titanium-nickel alloy) or alumina ceramic of comparable properties), or certain biocompatible polymer materials (e.g., polycarbonate, acrylonitrile butadiene styrene (ABS), high-density polyethylene (HDPE), polystyrene, polyether ether ketone (PEEK), polypropylene, urethane, teflon, polyethylene, polymethylmethacrylate, certain epoxies, silicone, or parylene and other polyesters as described above). On the other hand, an instrument intended for one-time use may be manufactured from a disposable polymer material, preferably one that degrades during autoclaving to ensure that the instrument is not used more than once (e.g., caprolactone, lactic acid, glycolic acid, acrylic, polycarbonate, or acrylonitrele butadiene styrene).

The external surfaces of the device case, surgical sizer, marking tool, and deployer are preferably non-abrasive and/or finished with smooth edges in order to prevent damage to the surrounding tissues contacted during implantation. In some embodiments, the instrument is surface-coated with parylene or a comparable hydrophobic material for an optimized smooth surface; surface coatings may be applied to both metal and disposable plastic tools. For example, the instrument may be injected-molded out of polymer (e.g. polycarbonate, ABS, HDPE, polystyrene or polypropylene) and then coated with Parylene C. Additionally, the underside of the instrument may be dipped in silicone or other materials commonly used by those skilled in the field to further optimize the surface. During the coating procedure, the groove, holes, or indentations in the portion of the shaft or handle or the marking portion may be used to hold the instrument so as to minimize the surface area that is not coated with conventional coating procedures.

Various aspects of the invention are described herein, but still others not described in detail but referred to can be found in Applicant's U.S. Patent Application Numbers: 10/486,408, entitled METHODS FOR CULTURE OF HESC ON FEEDER CELLS, filed August 6, 2002; 11/021,618, entitled DEFINITIVE ENDODERM, filed December 23, 2004; 11/115,868, entitled PDX1 EXPRESSING ENDODERM, filed April 26, 2005; 11/165,305, entitled METHODS FOR IDENTIFYING FACTORS FOR DIFFERENTIATING DEFINITIVE ENDODERM, filed June 23, 2005; 11/573,662, entitled METHODS FOR INCREASING DEFINITIVE ENDODERM DIFFERENTIATION OF PLURIPOTENT HUMAN EMBRYONIC STEM CELLS WITH PI-3 KINASE INHIBITORS, filed August 15, 2005; 12/729, 084 entitled PDX1-EXPRESSING DORSAL AND VENTRAL FOREGUT ENDODERM, filed October 27, 2005; 12/093,590, entitled MARKERS OF DEFINITIVE ENDODERM, filed November 14, 2005; 11/993,399, entitled EMBRYONIC STEM CELL CULTURE COMPOSITIONS AND METHODS OF USE THEREOF, filed June 20, 2006; 11/588,693, entitled PDX1-EXPRESSING DORSAL AND VENTRAL FOREGUT ENDODERM, filed October 27, 2006; 11/681,687, entitled ENDOCRINE PROGENITOR/PRECURSOR CELLS, PANCREATIC HORMONE-EXPRESSING CELLS AND METHODS OF PRODUCTION, filed March 2, 2007; 11/807,223, entitled METHODS FOR CULTURE AND PRODUCTION OF SINGLE CELL POPULATIONS OF HESC, filed May 24, 2007;11/773,944, entitled METHODS OF PRODUCING PANCREATIC HORMONES, filed July 5, 2007; 11/860,494, entitled METHODS FOR INCREASING DEFINITIVE ENDODERM PRODUCTION, filed September 24, 2007; 12/099,759, entitled METHODS OF PRODUCING PANCREATIC HORMONES, filed April 8, 2008; 12/107,020, entitled METHODS FOR PURIFYING ENDODERM AND PANCREATIC ENDODERM CELLS DERIVED FORM HUMAN EMBRYONIC STEM CELLS, filed April 21, 2008; 12/618,659, entitled ENCAPSULATION OF PANCREATIC LINEAGE CELLS DERIVED FROM HUMAN PLURIPOTENT STEM CELLS, filed November 13, 2009; 12/765,714 and 13/761,078, both entitled CELL COMPOSITIONS FROM DEDIFFERENTIATED REPROGRAMMED CELLS, filed April 22, 2010 and February 6, 2013; 11/838,054, entitled COMPOSITIONS AND METHODS USEFUL FOR CULTURING DIFFERENTIABLE CELLS, filed August 13, 2007; 12/264,760, entitled STEM CELL AGGREGATE SUSPENSION COMPOSITIONS AND METHODS OF DIFFERENTIATION THEREOF, filed November 4, 2008; 13/259,15, entitled SMALL MOLECULES SUPPORTING PLURIPOTENT CELL GROWTH, filed April 27, 2010; PCT/US11/25628, entitled LOADING SYSTEM FOR AN ENCAPSULATION DEVICE, filed February 21, 2011; 13/992,931, entitled AGENTS AND METHODS FOR INHIBITING PLURIPOTENT STEM CELLS, filed December 28, 2010; and U.S. Design Application Numbers: 29/408,366 filed December 12, 2011; 29/408,368 filed December 12, 2011; 29/423,365 filed May 31, 2012; and 29/447,944 filed March 13, 2013; and U.S. Provisional Application Numbers: 61/774,443, entitled SEMIPERMEABLE MACRO IMPLANTABLE CELLULAR ENCAPSULATION DEVICES, filed March 7, 2013; 61/775,480, entitled CRYOPRESERVATION, HIBERNATION AND ROOM TEMPERATURE STORAGE OF ENCAPSULATED PANCREATIC ENDODERM CELL AGGREGATES, filed March 8, 2013; 14/106,330, entitled IN VITRO DIFFERENTIATION OF PLURIPOTENT STEM CELLS TO PANCREATIC ENDODERM CELLS (PEC) AND ENDOCRINE CELLS, filed December 13, 2013; PCT/US2014/022065, entitled CRYOPRESERVATION, HIBERNATION AND ROOM TEMPERATURE STORAGE OF ENCAPSULATED PANCREATIC ENDODERM CELL, filed March 7, 2014; PCT/US2014/022109, SEMIPERMEABLE MACRO IMPLANTABLE CELLULAR ENCAPSULATION DEVICES, filed March 7, 2014; 14/201,630, filed March 7, 2014; and PCT/US2014/026529, IN VITRO DIFFERENTIATION OF PLURIPOTENT STEM CELLS TO PANCREATIC ENDODERM CELLS (PEC) AND ENDOCRINE CELLS, filed March 13, 2014.

## Claims

1. A device-fill pouch assembly (DFPA) (50) comprising a pouch (52, 54) for holding an implantable device with at least one port (202), wherein the pouch comprises a peelable first and second sheet (52, 54), **characterized in that** the pouch (52, 54) further comprises an inlet and outlet system (62, 66, 68, 72, 74), wherein the inlet system (62, 66, 68) extends interiorly into the pouch (52, 54) and is suitable for being detachably connected to (i) the implantable device port and (ii) a cell reservoir, and wherein the outlet system (72, 74) extends outside of the pouch (52, 54).

2. The DFPA (50) of claim 1, wherein the pouch (52, 54) holds an implantable device (200) with at least one port (202).

3. The DFPA (50) of claim 1 or 2, wherein the first sheet (52) is peelably adhered to the second sheet (54).

4. The DFPA (50) of any one of claims 1 to 3, wherein the DFPA (50) is wholly sterilizable, or comprised of a sterilizable material or paper.

5. The DFPA (50) of any one of claims 1 to 4, wherein the implantable device (200) is a semi-permeable macro-encapsulation device.

6. The DFPA (50) of any one of claims 1 to 5, wherein the implantable device (200) is inside a device case (1).

7. The DFPA (50) of claim 6, wherein the pouch (52, 54) is suitable for sterilizing the case (1) and implantable device (200).

8. The DFPA (50) of any one of claims 1 to 7, wherein the pouch (52, 54) is suitable for sterilizing and filling the implantable device (200).

9. The DFPA (50) of any one of claims 1 to 8, wherein the inlet system (62, 66, 68) comprises a spacer tube (62), and wherein the device port (202) of the implantable device (200) may be inserted into the spacer tube (62).

10. The DFPA (50) of any one of claims 1 to 9, wherein the inlet system (62, 66, 68) comprises a fluidic fitting (66, 68) and a spacer tube (62) affixed to the fluidic fitting (66, 68), wherein the spacer tube (62) is configured to receive the device port (202) of the implantable device (200).

11. The DFPA (50) of any one of claims 1 to 10 which is suitable for filling the implantable device (200) with living cells, preferably wherein the living cells are pancreatic progenitor cells, pancreatic and duodenal homeobox 1 (PDX1) positive pancreatic endoderm cells, endocrine precursor cells, or immature beta cells.

12. The DPFA (50) of any one of claims 1 to 11, wherein the implantable device (200) is filled with living pancreatic progenitor cells, PDX1 positive pancreatic endoderm cells, endocrine precursor cells, or immature beta cells.

13. The DFPA (50) of any one of claims 1 to 12, wherein the first sheet (52) comprises a peelable transparent window (58), and preferably wherein the transparent window (58) comprises a biaxially-oriented polyethylene terephthalate, a polyester film comprising stretched polyethylene terephthalate (PET), or a clear polymer film such as polyethylene.

14. The DFPA (50) of any one of claims 1 to 13, wherein (i) the first or second sheet (52, 54) comprises a sterilizable paper, preferably comprising polyester, polyvinyl chloride, polyethylene, polypropylene, polyolefin, an ethylene copolymer, ethylene-vinyl acetate (EVA), ethylene-methyl acrylate (EMA), ethylene-acrylic acid (EAA), an ionomer resin, a gas-permeable polyethylene fabric, a polypropylene non-woven fabric, a water-resistant fabric, a fabric resistant to chemical, or a fabric resistant to abrasion; or (ii) the DFPA (50) further comprises a thermoplastic transparent resin film between the first and second sheet.

## Patentansprüche

1. Beutelanordnung zur Befüllung einer Vorrichtung (device-fill pouch assembly; DFPA) (50), umfassend einen Beutel (52, 54) zur Aufnahme einer implantierbaren Vorrichtung mit mindestens einem Port (202), wobei der Beutel eine abziehbare erste und zweite Folie (sheet) (52, 54) umfasst, **dadurch gekennzeichnet, dass** der Beutel (52, 54) des Weiteren ein Einlass- und Auslasssystem (62, 66, 68, 72, 74) umfasst, wobei sich das Einlasssystem (62, 66, 68) innen in den Beutel (52, 54) erstreckt und geeignet ist, lösbar mit (i) dem Anschluss der implantierbaren Vorrichtung und (ii) einem Zellreservoir verbunden zu werden, und wobei sich das Auslasssystem (72, 74) außerhalb des Beutels (52, 54) erstreckt.

2. DFPA (50) nach Anspruch 1, wobei der Beutel (52, 54) eine implantierbare Vorrichtung (200) mit mindestens einem Port (202) aufnimmt.

3. DFPA (50) nach Anspruch 1 oder 2, wobei die erste Folie (52) abziehbar an die zweite Folie (54) adhäriert ist.

4. DFPA (50) nach einem der Ansprüche 1 bis 3, wobei das DFPA (50) vollständig sterilisierbar ist oder aus einem sterilisierbaren Material oder Papier besteht.

5. DFPA (50) nach einem der Ansprüche 1 bis 4, wobei die implantierbare Vorrichtung (200) eine semipermeable Makro-Verkapselungsvorrichtung ist.

6. DFPA (50) nach einem der Ansprüche 1 bis 5, wobei sich die implantierbare Vorrichtung (200) in einem Vorrichtungsgehäuse (1) befindet.

7. DFPA (50) nach Anspruch 6, wobei der Beutel (52, 54) zum Sterilisieren des Gehäuses (1) und der implantierbaren Vorrichtung (200) geeignet ist.

8. DFPA (50) nach einem der Ansprüche 1 bis 7, wobei der Beutel (52, 54) zum Sterilisieren und Befüllen der implantierbaren Vorrichtung (200) geeignet ist.

9. DFPA (50) nach einem der Ansprüche 1 bis 8, wobei das Einlasssystem (62, 66, 68) ein Abstandsröhrchen (spacer tube) (62) umfasst, und wobei der Vorrichtungs-Port (202) der implantierbaren Vorrichtung (200) in das Abstandsröhrchen (62) eingeführt werden kann.

10. DFPA (50) nach einem der Ansprüche 1 bis 9, wobei das Einlasssystem (62, 66, 68) eine Fluidverbindung (66, 68) und ein an der Fluidverbindung (66, 68) befestigtes Abstandsröhrchen (62) umfasst, wobei das Abstandsröhrchen (62) konfiguriert ist, den Vorrichtungs-Port (202) der implantierbaren Vorrichtung (200) aufzunehmen.

11. DFPA (50) nach einem der Ansprüche 1 bis 10, das für das Befüllen der implantierbaren Vorrichtung (200) mit lebenden Zellen geeignet ist, bevorzugt wobei die lebenden Zellen Pankreas-Vorläuferzellen, PDX1 (pancreatic and duodenal homeobox 1)-positive Endodermzellen des Pankreas, endokrine Vorläuferzellen oder unreife Beta-Zellen sind.

12. DFPA (50) nach einem der Ansprüche 1 bis 11, wobei die implantierbare Vorrichtung (200) mit lebenden Pankreas-Vorläuferzellen, PDX1-positiven Endodermzellen des Pankreas, endokrinen Vorläuferzellen oder unreifen Beta-Zellen befüllt ist.

13. DFPA (50) nach einem der Ansprüche 1 bis 12, wobei die erste Folie (52) ein abziehbares transparentes Fenster (58) umfasst, und bevorzugt wobei das transparente Fenster (58) ein biaxial orientiertes Polyethylenterephthalat, eine Polyesterfolie, die gestrecktes Polyethylenterephthalat (PET) umfasst, oder eine klare Polymerfolie wie Polyethylen umfasst.

14. DFPA (50) nach einem der Ansprüche 1 bis 13, wobei (i) die erste oder zweite Folie (52, 54) ein sterilisierbares Papier umfasst, das bevorzugt Polyester, Polyvinylchlorid, Polyethylen, Polypropylen, Polyolefin, ein Ethylencopolymer, Ethylenvinylacetat (EVA), Ethylenmethylacrylat (EMA), Ethylenacrylsäure (EAA), ein Ionomerharz, ein gasdurchlässiges Polyethylengewebe, einen Polypropylenvliesstoff (polypropylene non-woven fabric), ein wasserabweisendes Gewebe, ein chemikalienbeständiges Gewebe oder ein abriebfestes Gewebe umfasst; oder (ii) das DFPA (50) des Weiteren eine thermoplastische, transparente Harzfolie zwischen der ersten und der zweiten Folie umfasst.

## Revendications

1. Assemblage de poche de remplissage de dispositif (DFPA) (50) comprenant une poche (52, 54) destinée à contenir un dispositif implantable ayant au moins un orifice (202), dans lequel la poche comprend des première et deuxième feuilles pelables (52, 54), **caractérisé en ce que** la poche (52, 54) comprend en outre un système d'entrée et un système de sortie (62, 66, 68, 72, 74), dans lequel le système d'entrée (62, 66, 68) s'étend vers l'intérieur dans la poche (52, 54) et convient pour être raccordé de manière détachable à (i) l'orifice de dispositif implantable et (ii) un réservoir de cellules, et dans lequel le système de sortie (72, 74) s'étend à l'extérieur de la poche (52, 54).

2. DFPA (50) selon la revendication 1, dans lequel la poche (52, 54) contient un dispositif implantable (200) ayant au moins un orifice (202).

3. DFPA (50) selon la revendication 1 ou 2, dans lequel la première feuille (52) adhère en pouvant être pelée à la deuxième feuille (54).

4. DFPA (50) selon l'une quelconque des revendications 1 à 3, dans lequel le DFPA (50) est entièrement stérilisable, ou est composé de papier ou d'un matériau stérilisable.

5. DFPA (50) selon l'une quelconque des revendications 1 à 4, dans lequel le dispositif implantable (200) est un dispositif de macro-encapsulation semi-perméable.

6. DFPA (50) selon l'une quelconque des revendications 1 à 5, dans lequel le dispositif implantable (200) est à l'intérieur d'un boîtier de dispositif (1).

7. DFPA (50) selon la revendication 6, dans lequel la poche (52, 54) convient pour stériliser le boîtier (1) et le dispositif implantable (200).

8. DFPA (50) selon l'une quelconque des revendications 1 à 7, dans lequel la poche (52, 54) convient pour stériliser et remplir le dispositif implantable (200).

9. DFPA (50) selon l'une quelconque des revendications 1 à 8, dans lequel le système d'entrée (62, 66, 68) comprend un tube d'écartement (62), et dans lequel l'orifice de dispositif (202) du dispositif implantable (200) peut être inséré dans le tube d'écartement (62).

10. DFPA (50) selon l'une quelconque des revendications 1 à 9, dans lequel le système d'entrée (62, 66, 68) comprend un raccord fluidique (66, 68) et un tube d'écartement (62) fixé au raccord fluidique (66, 68), dans lequel le tube d'écartement (62) est configuré pour recevoir l'orifice de dispositif (202) du dispositif implantable (200).

11. DFPA (50) selon l'une quelconque des revendications 1 à 10, qui convient pour remplir le dispositif implantable (200) avec des cellules vivantes, de préférence dans lequel les cellules vivantes sont des cellules progénitrices pancréatiques, des cellules endodermiques pancréatiques positives à l'homéoboîte pancréatique et duodénale 1 (PDX1), des cellules précurseurs endocrines, ou des cellules bêta immatures.

12. DFPA (50) selon l'une quelconque des revendications 1 à 11, dans lequel le dispositif implantable (200) est rempli de cellules progénitrices pancréatiques vivantes, de cellules endodermiques pancréatiques PDX1-positives, de cellules précurseurs endocrines, ou de cellules bêta immatures.

13. DFPA (50) selon l'une quelconque des revendications 1 à 12, dans lequel la première feuille (52) comprend une fenêtre transparente pelable (58), et de préférence dans lequel la fenêtre transparente (58) comprend un poly(téréphtalate d'éthylène) à orientation biaxiale, un film de polyester comprenant un poly(téréphtalate d'éthylène) (PET) étiré, ou un film polymère incolore tel qu'un polyéthylène.

14. DFPA (50) selon l'une quelconque des revendications 1 à 13, dans lequel (i) la première ou deuxième feuille (52, 54) comprend un papier stérilisable, de préférence comprenant un polyester, un poly(chlorure de vinyle), un polyéthylène, un polypropylène, une polyoléfine, un copolymère d'éthylène, un éthylène-acétate de vinyle (EVA), un éthylène-acrylate de méthyle (EMA), un éthylène-acide acrylique (EAA), une résine ionomère, une étoffe de polyéthylène perméable aux gaz, une étoffe non tissée de polypropylène, une étoffe résistante à l'eau, une étoffe résistante aux produits chimiques, ou une étoffe résistante à l'abrasion ; ou (ii) le DFPA (50) comprend en outre un film de résine transparente thermoplastique entre les première et deuxième feuilles.
